# EUROPEAN PATENT APPLICATION

(11) **EP 3 916 088 A1**
(43) Date of publication of application: **01.12.2021**
(21) Application number: 20305559.5
(22) Date of filing: 28.05.2020
(51) Int. Cl.: C12N 9/64, G01N 33/569

(54) **FUSION PROTEINS COMPRISING THE SARS-COV-2 3CLPRO CATALYTIC DOMAIN AND THEIR USES FOR SCREENING ANTI-SARS-COV-2 AGENTS**

(71) Applicant: INSERM (Institut National de la Santé et de la Recherche Médicale), 75013 Paris (FR); Université de la Réunion Saint Denis, 97490 Saint Denis (FR); Institut De Recherche Pour Le Développement (IRD), 13002 Marseille 2 (FR); CENTRE NATIONAL DE LA RECHERCHE SCIENTIFIQUE -CNRS-, 75016 Paris (FR)
(72) Inventor: The designation of the inventor has not yet been filed
(74) Representative: Inserm Transfert

(57) **Abstract**

In late December 2019, a new betacoronavirus SARS-CoV-2 has emerged in Wuhan China. The World Health Organization has named the severe pneumonia caused by this new coronavirus COVID-19 (for Corona Virus Disease 2019, WHO, 2020). To fight against the COVID-19 pandemic in a long term, in addition to the containment measures implemented in many countries, several projects have been launched around the world to understand the viral evolution and the pathophysiological consequences of the infection in order to identify therapeutic targets and to implement innovative therapies. In particular, the 3C-like proteinase (3CLpro) of the severe acute respiratory syndrome coronavirus (SARS-CoV) plays a vital role in virus maturation and is proposed to be a key target for drug design against SARS. The present invention relates to fusion proteins comprising the SARS-CoV-2 3CLpro catalytic domain and their uses for screening anti-SARS-Cov-2 agents.

SEQUENCE LISTING

<110>  Inserm

<120>  FUSION PROTEINS COMPRISING THE SARS-COV-2 3CLPRO CATALYTIC DOMAIN
       AND THEIR USES FOR SCREENING ANTI-SARS-COV-2 AGENTS

<130>  BIO20244 GADEA / MC

<160>  12

<170>  PatentIn version 3.3

<210>  1
<211>  196
<212>  PRT
<213>  SARS-COV-2

<400>  1

Ser Gly Phe Arg Lys Met Ala Phe Pro Ser Gly Lys Val Glu Gly Cys
1               5                   10                  15

Met Val Gln Val Thr Cys Gly Thr Thr Thr Leu Asn Gly Leu Trp Leu
            20                  25                  30

Asp Asp Val Val Tyr Cys Pro Arg His Val Ile Cys Thr Ser Glu Asp
        35                  40                  45

Met Leu Asn Pro Asn Tyr Glu Asp Leu Leu Ile Arg Lys Ser Asn His
    50                  55                  60

Asn Phe Leu Val Gln Ala Gly Asn Val Gln Leu Arg Val Ile Gly His
65                  70                  75                  80

Ser Met Gln Asn Cys Val Leu Lys Leu Lys Val Asp Thr Ala Asn Pro
                85                  90                  95

Lys Thr Pro Lys Tyr Lys Phe Val Arg Ile Gln Pro Gly Gln Thr Phe
            100                 105                 110

Ser Val Leu Ala Cys Tyr Asn Gly Ser Pro Ser Gly Val Tyr Gln Cys
        115                 120                 125

Ala Met Arg Pro Asn Phe Thr Ile Lys Gly Ser Phe Leu Asn Gly Ser
    130                 135                 140

Cys Gly Ser Val Gly Phe Asn Ile Asp Tyr Asp Cys Val Ser Phe Cys
145                 150                 155                 160

Tyr Met His His Met Glu Leu Pro Thr Gly Val His Ala Gly Thr Asp
                165                 170                 175

Leu Glu Gly Asn Phe Tyr Gly Pro Phe Val Asp Arg Gln Thr Ala Gln
            180                 185                 190

Ala Ala Gly Thr
        195

<210>  2
<211>  10
<212>  PRT
<213>  Artificial

<220>
<223>  Myc Tag

<400>  2

Glu Gln Lys Leu Ile Ser Glu Glu Asp Leu
1               5                   10

<210>  3
<211>  8
<212>  PRT
<213>  Artificial

<220>
<223>  Strep Tag

<400>  3

Trp Ser His Pro Gln Phe Glu Lys
1               5

<210>  4
<211>  8
<212>  PRT
<213>  Artificial

<220>
<223>  Flag Tag

<400>  4

Asp Tyr Lys Asp Asp Asp Asp Lys
1               5

<210>  5
<211>  14
<212>  PRT
<213>  Artificial

<220>
<223>  V5 Tag

<400>  5

Gly Lys Pro Ile Pro Asn Pro Leu Leu Gly Leu Asp Ser Thr
1               5                   10

<210>  6
<211>  217
<212>  PRT
<213>  Schistosoma japonicum

<400>  6

Ser Pro Ile Leu Gly Tyr Trp Lys Ile Lys Gly Leu Val Gln Pro Thr
1               5                   10                  15

Arg Leu Leu Leu Glu Tyr Leu Glu Glu Lys Tyr Glu Glu His Leu Tyr
            20                  25                  30

Glu Arg Asp Glu Gly Asp Lys Trp Arg Asn Lys Lys Phe Glu Leu Gly
        35                  40                  45

Leu Glu Phe Pro Asn Leu Pro Tyr Tyr Ile Asp Gly Asp Val Lys Leu
    50                  55                  60

Thr Gln Ser Met Ala Ile Ile Arg Tyr Ile Ala Asp Lys His Asn Met
65                  70                  75                  80

Leu Gly Gly Cys Pro Lys Glu Arg Ala Glu Ile Ser Met Leu Glu Gly
                85                  90                  95

Ala Val Leu Asp Ile Arg Tyr Gly Val Ser Arg Ile Ala Tyr Ser Lys
            100                 105                 110

Asp Phe Glu Thr Leu Lys Val Asp Phe Leu Ser Lys Leu Pro Glu Met
        115                 120                 125

Leu Lys Met Phe Glu Asp Arg Leu Cys His Lys Thr Tyr Leu Asn Gly
    130                 135                 140

Asp His Val Thr His Pro Asp Phe Met Leu Tyr Asp Ala Leu Asp Val
145                 150                 155                 160

Val Leu Tyr Met Asp Pro Met Cys Leu Asp Ala Phe Pro Lys Leu Val
                165                 170                 175

Cys Phe Lys Lys Arg Ile Glu Ala Ile Pro Gln Ile Asp Lys Tyr Leu
            180                 185                 190

Lys Ser Ser Lys Tyr Ile Ala Trp Pro Leu Gln Gly Trp Gln Ala Thr
        195                 200                 205

Phe Gly Gly Gly Asp His Pro Pro Lys
    210                 215

<210>  7
<211>  7
<212>  PRT
<213>  Artificial

<220>
<223>  linker

<400>  7

Gly Gly Gly Ser Gly Gly Gly
1               5


<210>  8
<211>  8
<212>  PRT
<213>  Artificial

<220>
<223>  HRV 3C recognition site

<400>  8

Leu Glu Val Leu Pro Gln Gly Pro
1               5


<210>  9
<211>  450
<212>  PRT
<213>  Artificial

<220>
<223>  fusion protein as depicted in Figure 3

<400>  9

Met His His His His His His Gly Gly Gly Ser Gly Gly Gly Asp Tyr
1               5                   10                  15

Lys Asp Asp Asp Asp Lys Ser Pro Ile Leu Gly Tyr Trp Lys Ile Lys
            20                  25                  30

Gly Leu Val Gln Pro Thr Arg Leu Leu Leu Glu Tyr Leu Glu Glu Lys
        35                  40                  45

Tyr Glu Glu His Leu Tyr Glu Arg Asp Glu Gly Asp Lys Trp Arg Asn
    50                  55                  60

Lys Lys Phe Glu Leu Gly Leu Glu Phe Pro Asn Leu Pro Tyr Tyr Ile
65                  70                  75                  80

Asp Gly Asp Val Lys Leu Thr Gln Ser Met Ala Ile Ile Arg Tyr Ile
                85                  90                  95

Ala Asp Lys His Asn Met Leu Gly Gly Cys Pro Lys Glu Arg Ala Glu
            100                 105                 110

Ile Ser Met Leu Glu Gly Ala Val Leu Asp Ile Arg Tyr Gly Val Ser
        115                 120                 125

Arg Ile Ala Tyr Ser Lys Asp Phe Glu Thr Leu Lys Val Asp Phe Leu
    130                 135                 140

Ser Lys Leu Pro Glu Met Leu Lys Met Phe Glu Asp Arg Leu Cys His
145                 150                 155                 160

Lys Thr Tyr Leu Asn Gly Asp His Val Thr His Pro Asp Phe Met Leu
                165                 170                 175

Tyr Asp Ala Leu Asp Val Val Leu Tyr Met Asp Pro Met Cys Leu Asp
            180                 185                 190

Ala Phe Pro Lys Leu Val Cys Phe Lys Lys Arg Ile Glu Ala Ile Pro
        195                 200                 205

Gln Ile Asp Lys Tyr Leu Lys Ser Ser Lys Tyr Ile Ala Trp Pro Leu
    210                 215                 220

Gln Gly Trp Gln Ala Thr Phe Gly Gly Gly Asp His Pro Pro Lys Gly
225                 230                 235                 240

Gly Gly Ser Gly Gly Gly Leu Glu Val Leu Pro Gln Gly Pro Ser Gly
                245                 250                 255

Phe Arg Lys Met Ala Phe Pro Ser Gly Lys Val Glu Gly Cys Met Val
            260                 265                 270

Gln Val Thr Cys Gly Thr Thr Thr Leu Asn Gly Leu Trp Leu Asp Asp
        275                 280                 285

Val Val Tyr Cys Pro Arg His Val Ile Cys Thr Ser Glu Asp Met Leu
    290                 295                 300

Asn Pro Asn Tyr Glu Asp Leu Leu Ile Arg Lys Ser Asn His Asn Phe
305                 310                 315                 320

Leu Val Gln Ala Gly Asn Val Gln Leu Arg Val Ile Gly His Ser Met
                325                 330                 335

Gln Asn Cys Val Leu Lys Leu Lys Val Asp Thr Ala Asn Pro Lys Thr
            340                 345                 350

Pro Lys Tyr Lys Phe Val Arg Ile Gln Pro Gly Gln Thr Phe Ser Val
        355                 360                 365

Leu Ala Cys Tyr Asn Gly Ser Pro Ser Gly Val Tyr Gln Cys Ala Met
    370                 375                 380

Arg Pro Asn Phe Thr Ile Lys Gly Ser Phe Leu Asn Gly Ser Cys Gly
385                 390                 395                 400

Ser Val Gly Phe Asn Ile Asp Tyr Asp Cys Val Ser Phe Cys Tyr Met
                405                 410                 415

His His Met Glu Leu Pro Thr Gly Val His Ala Gly Thr Asp Leu Glu
            420                 425                 430

Gly Asn Phe Tyr Gly Pro Phe Val Asp Arg Gln Thr Ala Gln Ala Ala
        435                 440                 445

Gly Thr
    450

<210>  10
<211>  1367
<212>  DNA
<213>  Artificial

<220>
<223>  polynucleotide encoding the fusion protein as depicted in Figure
       3

<400>  10
catatgcatc atcatcatca tcatggtggt ggttccggtg gtggtgatta taaagatgat      60

gatgataaaa gcccgattct gggttattgg aaaattaaag gtctggttca gccgacgcgt     120

ctgctgctgg aatatctgga agaaaaatat gaagaacatc tgtatgaacg tgatgaaggt     180

gataaatggc gtaacaaaaa gtttgaactg ggtctggaat ttccgaatct gccgtattat     240

attgatggtg atgttaaact gacgcagagc atggcaatta ttcgttatat tgcagataaa     300

cataacatgc tgggcggttg tccgaaagaa cgtgcggaaa ttagcatgct ggaaggtgca     360

gttctggata ttcgttatgg tgttagccgc attgcctatt caaaagattt tgaaaccctg     420

aaagtggatt ttctgagtaa actgccggaa atgctgaaaa tgtttgaaga tcgtctgtgt     480

cataaaacct atctgaacgg tgatcatgtt acccatccgg attttatgct gtatgatgcc     540

ctggatgtgg ttctgtatat ggatccgatg tgtctggatg catttccgaa actggtttgt     600

tttaaaaagc gtattgaagc aatcccgcag attgataaat atctgaaaag cagcaaatat     660

atcgcgtggc cgctgcaggg ttggcaggcc acttttggtg gtggtgatca tccgccgaaa     720

ggtggcggta gcggtggtgg tctggaagtt ctgccgcagg gtccgagtgg ttttagaaaa 780

atggcattcc catctggtaa agttgagggt tgtatggtac aagtaacttg tggtacaact 840

acacttaacg gtctttggct tgatgacgta gtttactgtc caagacatgt gatctgcacc 900

tctgaagaca tgcttaaccc taattatgaa gatttactca ttcgtaagtc taatcataat 960

ttcttggtac aggctggtaa tgttcaactc agggttattg gacattctat gcaaaattgt 1020

gtacttaagc ttaaggttga tacagccaat cctaagacac ctaagtataa gtttgttcgc 1080

attcaaccag gacagacttt ttcagtgtta gcttgttaca atggttcacc atctggtgtt 1140

taccaatgtg ctatgaggcc caatttcact attaagggtt cattccttaa tggttcatgt 1200

ggtagtgttg gttttaacat agattatgac tgtgtctctt tttgttacat gcaccatatg 1260

gaattaccaa ctggagttca tgctggcaca gacttagaag gtaactttta tggacctttt 1320

gttgacaggc aaacagcaca agcagctggt acgtgataag cggccgc 1367

<210> 11
<211> 6
<212> DNA
<213> Artificial

<220>
<223> Nde I site

<400> 11
catatg 6

<210> 12
<211> 14
<212> DNA
<213> Artificial

<220>
<223> Not I sequence

<400> 12
tgataagcgg ccgc 14

## Description

### FIELD OF THE INVENTION:

The invention is in the field of medicine and in particular virology.

### BACKGROUND OF THE INVENTION:

*Coronaviridae* is a family of enveloped, positive-sense, single-stranded RNA viruses. The viral genome is 26-32 kilobases in length. The particles are typically decorated with large (∼20 nm), club- or petal-shaped surface projections (the "peplomers" or "spikes"), which in electron micrographs of spherical particles create an image reminiscent of the solar corona. In late December 2019, a new betacoronavirus SARS-CoV-2 has emerged in Wuhan China [1-3]. The World Health Organization has named the severe pneumonia caused by this new coronavirus COVID-19 (for Corona Virus Disease 2019, WHO, 2020). Since its emergence, the SARS-CoV-2 has spread to 159 countries across the five continents causing, at the time of the writing, about 213,254 human infections with 81,238 cases in China (ECDC, March 19 2020). Europe has recently become the epicenter of COVID-19 epidemics with 82,869 confirmed cases; the majority of them being reported in Italy with 35,713 cases and 2978 deaths. In France, the number of confirmed cases is increasing with about 10,995 and 372 deaths on mid-march 2019 (Santé Publique France). To fight against the COVID-19 pandemic in a long term, in addition to the containment measures implemented in many countries, several projects have been launched around the world to understand the viral evolution and the pathophysiological consequences of the infection in order to identify therapeutic targets and to implement innovative therapies. In particular, the 3C-like proteinase (3CLpro) of the severe acute respiratory syndrome coronavirus (SARS-CoV) plays a vital role in virus maturation and is proposed to be a key target for drug design against SARS.

### SUMMARY OF THE INVENTION:

As defined by the claims, the invention relates to fusion proteins comprising the SARS-CoV-2 3CLpro catalytic domain and their uses for screening anti-SARS-Cov-2 agents.

### DETAILED DESCRIPTION OF THE INVENTION:

### Definitions:

As used herein, the term **"subject"** or **"subject in need thereof',** is intended for a human or non-human mammal. Typically the patient is affected or likely to be infected with SARS-Cov-2.

As used herein, the term **"coronavirus"** has its general meaning in the art and refers to any member of members of the *Coronaviridae* family. Coronavirus is a virus whose genome is plus-stranded RNA of about 27 kb to about 33 kb in length depending on the particular virus. The virion RNA has a cap at the 5' end and a poly A tail at the 3' end. The length of the RNA makes coronaviruses the largest of the RNA virus genomes. In particular, coronavirus RNAs encode: (1) an RNA-dependent RNA polymerase; (2) N-protein; (3) three envelope glycoproteins; plus (4) three non-structural proteins. These coronaviruses infect a variety of mammals and birds. They cause respiratory infections (common), enteric infections (mostly in infants >12 mo.), and possibly neurological syndromes. Coronaviruses are transmitted by aerosols of respiratory secretions.

As used herein, the term **"Severe Acute Respiratory Syndrome coronavirus 2"** or **"SARS-Cov-2"** has its general meaning in the art and refers to the strain of coronavirus that causes coronavirus disease 2019 (COVID-19), a respiratory syndrome that manifests a clinical pathology resembling mild upper respiratory tract disease (common cold-like symptoms) and occasionally severe lower respiratory tract illness and extra-pulmonary manifestations leading to multi-organ failure and death. In particular, the term refers to the severe acute respiratory syndrome coronavirus 2 isolate 2019-nCoV_HKU-SZ-005b_2020 for which the complete genome is accessible under the NCBI access number MN975262.

As used herein, the term **"Covid-19"** refers to the respiratory disease induced by the Severe Acute Respiratory Syndrome coronavirus 2.

As used herein, the term **"3C-like proteinase"** or **"3CLpro"** refers to a SARS-Cov-2 protease that plays a vital role in virus maturation. In particular, the protease cleaves 11 sites in the SARS-Cov-2 polyproteins to generate individual functional proteins, including an RNA-dependent RNA polymerase, a helicase, a single-stranded RNA-binding protein, an exoribonuclease, an endoribonuclease, and a 2' -O-ribose methyltransferase. 3CLpro is a cysteine protease that is excised from polyproteins by its own proteolytic activity and forms a homodimer with one active site per subunit. The amino acid sequence of 3CLpro is represented by SEQ ID NO: 1.

SEQ ID NO:1 Sequence of SARS-CoV-2 nsp5-pp1a/pp1ab 3CL (chymotrypsin-
like) protease (3CLpro) catalytic domain (1-196) obtained from NCBI
database (NC_045512.2)
SGFRKMAFPSGKVEGCMVQVTCGTTTLNGLWLDDVVYCPRHVICTSEDMLNPNYEDLLIRKSNHNFLVQ
AGNVQLRVIGHSMQNCVLKLKVDTANPKTPKYKFVRIQPGQTFSVLACYNGSPSGVYQCAMRPNFTIKG
SFLNGSCGSVGFNIDYDCVSFCYMHHMELPTGVHAGTDLEGNFYGPFVDRQTAQAAGT

As used herein, the term **"catalytic domain of the 3CLpro"** refers to the amino acid sequence that ranges from the amino acid residue (S) at position 1 to the amino acid residue (T) at position 196 in SEQ ID NO:1.

As used herein, the terms **"polypeptide", "peptide",** and **"protein"** are used interchangeably herein to refer to polymers of amino acids of any length. The terms also encompass an amino acid polymer that has been modified; for example, disulfide bond formation, glycosylation, lipidation, phosphorylation, or conjugation with a labeling component. Polypeptides when discussed in the context of gene therapy refer to the respective intact polypeptide, or any fragment or genetically engineered derivative thereof, which retains the desired biochemical function of the intact protein.

As used herein, the term **"polynucleotide"** refers to a polymeric form of nucleotides of any length, including deoxyribonucleotides or ribonucleotides, or analogs thereof. A polynucleotide may comprise modified nucleotides, such as methylated nucleotides and nucleotide analogs, and may be interrupted by non-nucleotide components. If present, modifications to the nucleotide structure may be imparted before or after assembly of the polymer. The term polynucleotide, as used herein, refers interchangeably to double- and single-stranded molecules. Unless otherwise specified or required, any embodiment of the invention described herein that is a polynucleotide encompasses both the double-stranded form and each of two complementary single-stranded forms known or predicted to make up the double-stranded form.

As used herein, the term **"codon-optimized"** refers to polynucleotide sequence that has been optimized to increase expression in host cell by substituting one or more codons normally present in a coding sequence with a codon for the same (synonymous) amino acid. In this manner, the protein encoded by the gene is identical, but the underlying nucleobase sequence of the gene or corresponding mRNA is different. In some embodiments, the optimization substitutes one or more rare codons (that is, codons for tRNA that occur relatively infrequently in cells from a particular species) with synonymous codons that occur more frequently to improve the efficiency of translation. For example, in codon-optimization one or more codons in a coding sequence are replaced by codons that occur more frequently in host cells for the same amino acid. Desirably, a codon-optimized gene exhibits improved protein expression, for example, the protein encoded thereby is expressed at a detectably greater level in a cell compared with the level of expression of the protein provided by the wildtype gene in an otherwise similar cell.

As used herein, the term **"identity"** refers to an exact nucleotide to nucleotide or amino acid to amino acid correspondence of two polynucleotides or polypeptide sequences, respectively. Percent identity can be determined by a direct comparison of the sequence information between two molecules by aligning the sequences, counting the exact number of matches between the two aligned sequences, dividing by the length of the shorter sequence, and multiplying the result by 100. According to the invention a first amino acid sequence having at least 90% of identity with a second amino acid sequence means that the first sequence has 90; 91; 92; 93; 94; 95; 96; 97; 98; 99 or 100% of identity with the second amino acid sequence. Sequence identity is frequently measured in terms of percentage identity (or similarity or homology); the higher the percentage, the more similar are the two sequences. Methods of alignment of sequences for comparison are well known in the art. Various programs and alignment algorithms are described in: Smith and Waterman, Adv. Appl. Math., 2:482, 1981; Needleman and Wunsch, J. Mol. Biol., 48:443, 1970; Pearson and Lipman, Proc. Natl. Acad. Sci. U.S.A., 85:2444, 1988; Higgins and Sharp, Gene, 73:237-244, 1988; Higgins and Sharp, CABIOS, 5:151-153, 1989; Corpet et al. Nuc. Acids Res., 16:10881-10890, 1988; Huang et al., Comp. Appls Biosci., 8:155-165, 1992; and Pearson et al., Meth. Mol. Biol., 24:307-31, 1994). Altschul et al., Nat. Genet., 6:119-129, 1994, presents a detailed consideration of sequence alignment methods and homology calculations. By way of example, the alignment tools ALIGN (Myers and Miller, CABIOS 4:11-17, 1989) or LFASTA (Pearson and Lipman, 1988) may be used to perform sequence comparisons (Internet Program® 1996, W. R. Pearson and the University of Virginia, fasta20u63 version 2.0u63, release date December 1996). ALIGN compares entire sequences against one another, while LFASTA compares regions of local similarity. These alignment tools and their respective tutorials are available on the Internet at the NCSA Website, for instance. Alternatively, for comparisons of amino acid sequences of greater than about 30 amino acids, the Blast 2 sequences function can be employed using the default BLOSUM62 matrix set to default parameters, (gap existence cost of 11, and a per residue gap cost of 1). When aligning short peptides (fewer than around 30 amino acids), the alignment should be performed using the Blast 2 sequences function, employing the PAM30 matrix set to default parameters (open gap 9, extension gap 1 penalties). The BLAST sequence comparison system is available, for instance, from the NCBI web site; see also Altschul et al., J. Mol. Biol., 215:403-410, 1990; Gish. & States, Nature Genet., 3:266-272, 1993; Madden et al. Meth. Enzymol., 266:131-141, 1996; Altschul et al., Nucleic Acids Res., 25:3389-3402, 1997; and Zhang & Madden, Genome Res., 7:649-656, 1997.

As used herein, the expression **"derived from"** refers to a process whereby a first component (e.g., a first polypeptide), or information from that first component, is used to isolate, derive or make a different second component (e.g., a second polypeptide that is different from the first one).

As used herein, the term **"tag"** refers to a polypeptide sequence that can be attached to a second polypeptide. Typically, the tag is an expression tag, a purification tag or both.

As used herein, the term **"expression tag"** refers to any polypeptide that can be attached to a second polypeptide and is supposed to support the solubility, stability and/or the expression of a recombinant polypeptide of interest, such as fusion protein of the invention.

As used herein, the term **"purification tag"** refers to any polypeptide sequence suitable for purification or identification of a recombinant polypeptide such as fusion protein of the invention. The purification tag specifically binds to another moiety with affinity for the purification tag. Such moieties which specifically bind to a purification tag are usually attached to a matrix or a resin, such as agarose beads. Moieties which specifically bind to purification tags include antibodies, other proteins (e.g. Protein A or Streptavidin), nickel or cobalt ions or resins, biotin, amylose, maltose, and cyclodextrin. Exemplary purification tags include histidine (HIS) tags (such as a hexahistidine peptide), which will bind to metal ions such as nickel or cobalt ions. Other exemplary purification tags are the myc tag (SEQ ID NO:2 EQKLISEEDL), the Strep tag (SEQ ID NO:3 WSHPQFEK), the Flag tag (SEQ ID NO:4 DYKDDDDK) and the V5 tag (SEQ ID NO:5 GKPIPNPLLGLDST).

As used herein, the term **"glutathione S-transferase tag"** or **"GST tag"** is derived from an enzyme found in eukaryotes and prokaryotes. A GST tag may be the full length, a portion, or a modified version of the wild-type *E. coli* glutathione S-transferase protein, so long as the presence of the GST tag increases the solubility of the resulting recombinant fusion protein and confers to the fusion protein the binding affinity to glutathione. An exemplary GST sequence is set forth in SEQ ID NO:6.

SEQ ID NO:6 (Glutathione S-transferase class-mu 26 kDa isozyme, from
Schistosoma japonicum (UniProtKB - P08515)
SPILGYWKIKGLVQPTRLLLEYLEEKYEEHLYERDEGDKWRNKKFELGLEFPNLPYYIDGDVKLTQSMA
IIRYIADKHNMLGGCPKERAEISMLEGAVLDIRYGVSRIAYSKDFETLKVDFLSKLPEMLKMFEDRLCH
KTYLNGDHVTHPDFMLYDALDVVLYMDPMCLDAFPKLVCFKKRIEAIPQIDKYLKSSKYIAWPLQGWQA
TFGGGDHPPK

As used herein, the term **"maltose binding protein tag"** or **"MBP tag"** is derived from an *Escherichia coli* protein involved in the maltose/maltodextrin system. An MBP tag may be the full length, a portion, or a modified version of the wild-type E. coli maltose binding protein, so long as the presence of the MBP tag increases the solubility of the resulting recombinant fusion protein and confers to the fusion protein the binding affinity to amylose. An exemplary MBP sequence is set forth in GenBank Accession No. NP 418458.1.

As used herein, the term **"fusion protein"** refers to a single polypeptide chain having at least two polypeptide domains that are not normally present in a single, natural polypeptide. Thus, naturally occurring proteins are not "fusion proteins", as used herein. Preferably, a polypeptide of interest such as the catalytic domain of the 3CLpro is fused with at least one polypeptide domain (e.g. an expression Tag) via a peptide bond and the fusion protein may also include the linking regions of amino acids between amino acid portions derived from separate proteins. The polypeptide domain fused to the polypeptide of interest may enhance solubility and/or expression of the polypeptide of interest and may also provide a purification tag to allow purification of the recombinant fusion protein from the host cell or culture supernatant, or both.

As used herein, the term **"linker"** refers to a sequence of at least one amino acid that links the polypeptide of the invention to the expression tag. Such a linker may be useful to prevent steric hindrances. Typically a linker comprises 2, 3; 4; 5; 6; 7; 8; 9; 10; 11; 12; 13; 14; 15; 16; 17; 18; 19; or 20 amino acids.

As used herein, the term **"cleavable sequence"** refers to an amino acid sequence that comprises a cleavage site. The term **"cleavage site"** refers to the bond (e.g. a scissile bond) cleaved by an agent. A cleavage site for a protease includes the specific amino acid sequence recognized by the protease during proteolytic cleavage and typically includes the surrounding one to six amino acids on either side of the scissile bond, which bind to the active site of the protease and are needed for recognition as a substrate. The cleavable sequence may include a cleavage site specific for an enzyme, such as a protease or other cleavage agent. A cleavable sequence is typically cleavable under physiological conditions.

As used herein, the term **"recombinant"** refers to an artificial combination of two otherwise separated segments of sequence, e.g., by chemical synthesis or by the manipulation of isolated segments of amino acids or of nucleic acids by genetic engineering techniques.

As used herein, the term **"vector"** refers to an agent that is capable of transferring nucleic acid sequences to target cells (e.g., viral vectors, non-viral vectors, particulate carriers, and liposomes). Typically, "vector construct," "expression vector, " and "gene transfer vector," mean any nucleic acid construct capable of directing the expression of a nucleic acid of interest and which can transfer nucleic acid sequences to target cells. Thus, the term includes cloning and expression vehicles, as well as viral vectors.

As used herein, the term **"host cell"** may be any of a number commonly used cells in the production of exogenous polypeptides or proteins, including eukaryotic and prokaryotic host cells.

As used herein, the term **"transfection"** refers to a wide variety of techniques commonly used for the introduction of exogenous DNA into a prokaryotic or eukaryotic host cell, e.g., electroporation, calcium-phosphate precipitation, DEAE-dextran transfection and the like. The host cell may be **"transfected"** with the vector of the invention by any conventional means known to the skilled artisan. For example transfection may be a transient transfection.

As used herein, the term **"label"** refers to a composition that is detectable with or without instrumentation, for example, by visual inspection, spectroscopy, or a photochemical, biochemical, immunochemical or chemical reaction. Exemplary labels (non-limiting) include fluorescent dyes and proteins, electron-dense reagents, enzymes (such as those commonly used in an ELISA), and binding labels or tags, such as biotin, digoxigenin, or other haptens or peptides for an antiserum or antibody. For example, a label can generate a measurable signal such as fluorescent light in a sample.

As used herein, the term **"solid support"** or **"solid carrier"** may be any solid or semisolid insoluble surface suitable for immobilizing the fusion protein of the invention. Attachment of the fusion protein to the solid support can be covalent or non-covalent. Non-covalent interactions can be mediated by electrostatic, π-effects, van der Waals forces, and hydrophobic effects. Covalent attachment is mediated through a chemical reaction between reactive groups on the surface of the solid support and the fusion protein (for example, biotinylated fusion protein) of the invention which form a covalent bond.

As used herein, the term **"treatment"** or **"treat"** refer to both prophylactic or preventive treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a patient having a medical disorder or who ultimately may acquire the disorder, in order to prevent, cure, delay the onset of, reduce the severity of, or ameliorate one or more symptoms of a disorder or recurring disorder, or in order to prolong the survival of a patient beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at a regular interval, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

### Fusions proteins:

One object of the invention relates to a recombinant fusion protein wherein the catalytic domain of the SAR-Cov-2 3CLpro fused to one or more expression tag(s).

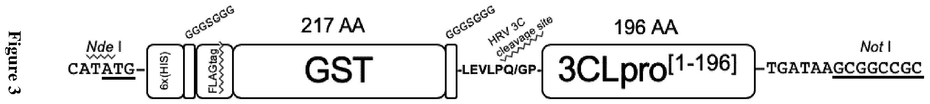

In some embodiments, the catalytic domain of the SARS-Cov-2 3CLpro is fused to one or more GST-Tag or MBP tag. In some embodiments, the catalytic domain of the SARS-Cov-2 3CLpro is fused to one GST-Tag as depicted in **Figure 3****.**

In some embodiments, the catalytic domain of the SARS-Cov-2 3CLpro is fused either directly or via a linker at its C-terminal end to the N-terminal end of the expression tag, or at its N-terminal end to the C-terminal end of the expression tag. As used herein, the term "directly" means that the (first or last) amino acid at the terminal end (N or C-terminal end) of the polypeptide of the invention is fused to the (first or last) amino acid at the terminal end (N or C-terminal end) of the expression tag. In other words, in this embodiment, the last amino acid of the C-terminal end of said polypeptide is directly linked by a covalent bond to the first amino acid of the N-terminal end of said expression tag, or the first amino acid of the N-terminal end of said polypeptide is directly linked by a covalent bond to the last amino acid of the C-terminal end of said expression tag.

The linker sequence may be a naturally occurring sequence or a non-naturally occurring sequence. One useful group of linker sequences are linkers derived from the hinge region of heavy chain antibodies as described in WO 96/34103 and WO 94/04678. Other examples are poly-alanine linker sequences such as Ala-Ala-Ala. Further preferred examples of linker sequences are Gly/Ser linkers of different length including the linker having the amino acid sequence as set forth in SEQ ID NO:7 (GGGSGGG).

In some embodiments, the fusion protein of the invention further comprises one or more purification tag. In some embodiments, the fusion protein of the invention comprises a 6-Histidine (HIS) tag and a Flag tag as depicted in **Figure 3****.**

In some embodiments, the fusion protein of the invention comprises a cleavable sequence. In some embodiments, the cleavable sequence is inserted between the catalytic domain of the 3CLpro and the expression tag (e.g. GST tag). The cleavable sequence is designed for selective cleavage by a particular protease. The cleavage site of the cleavable sequence includes the specific amino acid sequence recognized by the protease during proteolytic cleavage and typically includes the surrounding one to six amino acids on either side of the scissile bond, which bind to the active site of the protease and are needed for recognition as a substrate. The cleavable sequence may contain any protease recognition motif known in the art and is typically cleavable under physiological conditions. In some embodiments, the cleavable sequence comprises the HRV 3C recognition site as set forth in SEQ ID NO:8 (LEVLPQGP).

In some embodiments, the catalytic domain of the 3CLpro is preceded by a 6-Histidine (HIS) tag, a linker (GGGSGGG), a FLAG tag and a glutathione S-transferase (GST) tag. In some embodiments, the catalytic domain of the 3CLpro is fused to the GST tag by the linker (GGGSGGG) and a HRV 3C cleavage sequence.

In some embodiments, the fusion protein of the invention consists of the polypeptide depicted in **Figure 3**. In some embodiments, the fusion protein of the invention consists of an amino acid sequence having at least 90% of identity with the amino acid sequence as set forth in SEQ ID NO:9.

SEQ ID NO:9 (full sequence of the fusion protein according to Figure
3)
MHHHHHHGGGSGGGDYKDDDDKSPILGYWKIKGLVQPTRLLLEYLEEKYEEHLYERDEGDKWRNKKFEL
GLEFPNLPYYIDGDVKLTQSMAIIRYIADKHNMLGGCPKERAEISMLEGAVLDIRYGVSRIAYSKDFET
LKVDFLSKLPEMLKMFEDRLCHKTYLNGDHVTHPDFMLYDALDVVLYMDPMCLDAFPKLVCFKKRIEAI
PQIDKYLKSSKYIAWPLQGWQATFGGGDHPPKGGGSGGGLEVLPQGPSGFRKMAFPSGKVEGCMVQVTC

GTTTLNGLWLDDVVYCPRHVICTSEDMLNPNYEDLLIRKSNHNFLVQAGNVQLRVIGHSMQNCVLKLKV
DTANPKTPKYKFVRIQPGQTFSVLACYNGSPSGVYQCAMRPNFTIKGSFLNGSCGSVGFNIDYDCVSFC
YMHHMELPTGVHAGTDLEGNFYGPFVDRQTAQAAGT

### Polynucleotides, vectors and host cells:

A further object of the invention relates to a polynucleotide that encodes for the fusion protein of the invention.

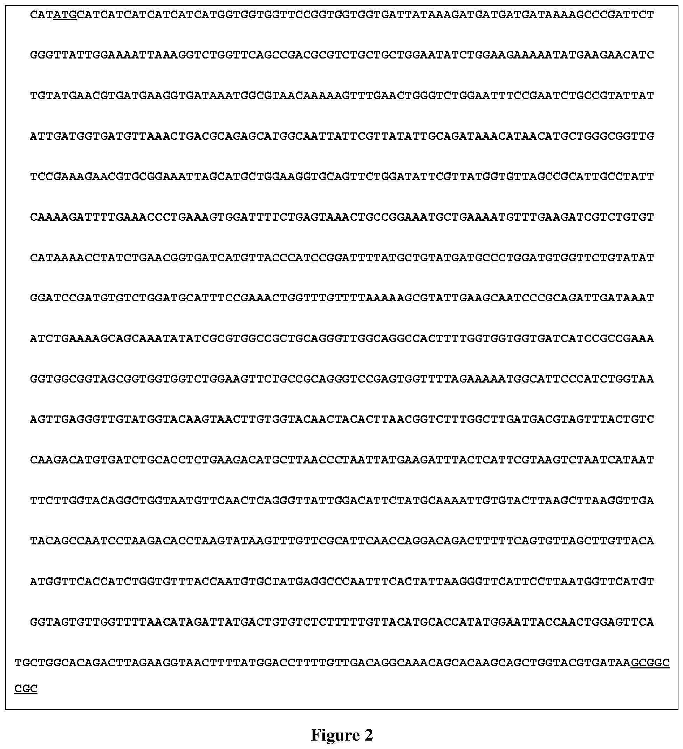

In some embodiments, the polynucleotide of the invention is codon-optimized. In some embodiments, the polynucleotide of the invention comprises a nucleic acid sequence having at least 90% of identify with the nucleic acid sequence as set forth in SEQ ID NO:10. In some embodiments, the polynucleotide of the invention comprises the nucleic acid sequence of **Figure 2**.

SEQ ID NO:10 (nucleotide sequence of SARS-CoV-2 3CLpro (1-196) bacterial expression construct. The start codon is followed in frame by a 6-HIS tag, a GGGSGGG linker, FLAG- and GST-tags, a second GGGSGGG linker, a HRV 3C cleavage site and the 3CLpro(1-196) catalytic domain. The 3CLpro(1-196) catalytic domain has been codon optimized for bacterial expression in E.coli)
CATATGCATCATCATCATCATCATGGTGGTGGTTCCGGTGGTGGTGATTATAAAGATGATGATGATAAA
AGCCCGATTCTGGGTTATTGGAAAATTAAAGGTCTGGTTCAGCCGACGCGTCTGCTGCTGGAATATCTG
GAAGAAAAATATGAAGAACATCTGTATGAACGTGATGAAGGTGATAAATGGCGTAACAAAAAGTTTGAA
CTGGGTCTGGAATTTCCGAATCTGCCGTATTATATTGATGGTGATGTTAAACTGACGCAGAGCATGGCA
ATTATTCGTTATATTGCAGATAAACATAACATGCTGGGCGGTTGTCCGAAAGAACGTGCGGAAATTAGC
ATGCTGGAAGGTGCAGTTCTGGATATTCGTTATGGTGTTAGCCGCATTGCCTATTCAAAAGATTTTGAA
ACCCTGAAAGTGGATTTTCTGAGTAAACTGCCGGAAATGCTGAAAATGTTTGAAGATCGTCTGTGTCAT
AAAACCTATCTGAACGGTGATCATGTTACCCATCCGGATTTTATGCTGTATGATGCCCTGGATGTGGTT
CTGTATATGGATCCGATGTGTCTGGATGCATTTCCGAAACTGGTTTGTTTTAAAAAGCGTATTGAAGCA
ATCCCGCAGATTGATAAATATCTGAAAAGCAGCAAATATATCGCGTGGCCGCTGCAGGGTTGGCAGGCC
ACTTTTGGTGGTGGTGATCATCCGCCGAAAGGTGGCGGTAGCGGTGGTGGTCTGGAAGTTCTGCCGCAG
GGTCCGAGTGGTTTTAGAAAAATGGCATTCCCATCTGGTAAAGTTGAGGGTTGTATGGTACAAGTAACT
TGTGGTACAACTACACTTAACGGTCTTTGGCTTGATGACGTAGTTTACTGTCCAAGACATGTGATCTGC
ACCTCTGAAGACATGCTTAACCCTAATTATGAAGATTTACTCATTCGTAAGTCTAATCATAATTTCTTG
GTACAGGCTGGTAATGTTCAACTCAGGGTTATTGGACATTCTATGCAAAATTGTGTACTTAAGCTTAAG
GTTGATACAGCCAATCCTAAGACACCTAAGTATAAGTTTGTTCGCATTCAACCAGGACAGACTTTTTCA
GTGTTAGCTTGTTACAATGGTTCACCATCTGGTGTTTACCAATGTGCTATGAGGCCCAATTTCACTATT

AAGGGTTCATTCCTTAATGGTTCATGTGGTAGTGTTGGTTTTAACATAGATTATGACTGTGTCTCTTTT
TGTTACATGCACCATATGGAATTACCAACTGGAGTTCATGCTGGCACAGACTTAGAAGGTAACTTTTAT
GGACCTTTTGTTGACAGGCAAACAGCACAAGCAGCTGGTACGTGATAAGCGGCCGC

In some embodiment, the polynucleotide is included in a suitable vector, such as a plasmid, cosmid, episome, artificial chromosome, phage or viral vector. So, a further object of the invention relates to a vector in which the polynucleotide of the invention is associated with suitable elements for controlling transcription (in particular promoter, enhancer and, optionally, terminator) and, optionally translation, and also the recombinant vectors into which a nucleic acid in accordance with the invention is inserted. These recombinant vectors may, for example, be cloning vectors, or expression vectors. Examples of suitable vectors include plasmids, typically replicating plasmids comprising an origin of replication, or integrative plasmids, such as for instance pUC, pcDNA, pBR, and the like.

A further aspect of the invention relates to a host cell comprising a polynucleotide encoding for the fusion protein of the invention. In particular, a subject of the invention is a prokaryotic host cell (e.g. *E. coli*) genetically transformed with at least one polynucleotide of the invention. In some embodiments, for expressing and producing fusion proteins of the invention, prokaryotic cells, in particular *E. coli* cells, will be chosen. Prokaryotic cells have the advantages to produce protein in large amounts. The construction of expression vectors in accordance with the invention, and the transformation of the host cells can be carried out using conventional molecular biology techniques. The fusion protein of the invention, can, for example, be obtained by culturing genetically transformed cells in accordance with the invention and recovering the fusion protein expressed by said cell, from the culture. They may then, if necessary, be purified by conventional procedures, known in themselves to those skilled in the art, for example by fractional precipitation, in particular ammonium sulfate precipitation, electrophoresis, gel filtration, affinity chromatography, etc. In particular, conventional methods for preparing and purifying recombinant proteins may be used for producing the fusion protein of the invention in accordance with the invention. In particular, antibodies having specificities for the purification tags may be used for the purification steps.

### Screening methods:

The fusion protein of the invention is particularly suitable for preparing assays suitable for screening a plurality of test substances for their ability to inhibit the protease activity of the catalytic domain of the 3CLpro. Selected test substances may be then used as anti-viral compounds that could be used for the treatment of COVID-19.

In some embodiments, once the fusion protein of the present invention is purified, the catalytic domain of the 3CLpro is released by using a protease that cleaves the inserted cleavable sequence. Then the catalytic domain is used for the screening purposes.

In some embodiments, the fusion protein of the present invention is immobilized in a solid support for e.g. allowing high-throughput screening of protease inhibitors. The solid support is typically substantially insoluble in aqueous phases. A large number of solid supports is available and is known to one of ordinary skill in the art. Solid supports that may be employed in accordance with the invention include beads, plates, microtiter plates, filter material, sheets, microchips, wires, microcapillaries, hydrogels, filaments, fibers, to name a few. The form or shape of the solid support may vary, depending on the application. Suitable examples include, but are not limited to, nanoparticles or microparticles of various sizes, microtiter plates of any dimension, gels (aerogel, hydrogel, resin), wires, filaments, slides, strips, microchips, wells, fibers, and combinations thereof. The solid material can be magnetic or non-magnetic. Most preferred solid supports are microparticles such as beads (for example, streptavidin beads, thiol-activated beads, avidin beads, or maleimide beads) or plates (for example, streptavidin/avidin coated plates or maleimide activated plates).

In particular, the fusion protein of the invention is particular suitable for preparing a protease assay so as to provide a rapid and specific identification of protease inhibitors for inhibiting the replication of SARS-Cov-2. For instance, in this assay system, substrates are labelled with a detectable molecule (e.g. fluorescent label). Once substrates are cleaved by the catalytic domain of the 3CLpro, the label can be detected. Addition of protease inhibitors to the assay inhibits the cleavage of the substrates, which leads to reduced intensity of the label, enabling screening of potential protease inhibitors.

The test substance of the invention may be selected from a library of substances previously synthesised, or a library of substances for which the structure is determined in a database, or from a library of substances that have been synthesised de novo. The test substance may be selected from the group of (a) proteins or peptides, (b) nucleic acids and (c) organic or chemical substances.

In some embodiments, the screening method of the invention further comprises the step consisting in determining whether the selected substance selected inhibits the replication of SARS-Cov-2 in a host cell and a step that consists in positively selecting the test substance capable of inhibiting the replication of said SARS-Cov-2 in said host cell. In some embodiments, the method comprises the steps consisting of i) infecting said host cell with said SARS-Cov-2 and ii) culturing said infected cell in presence of the test substance, iii) comparing the replicating capacity of the virus with the replication capacity determined in the absence of the test substance and iv) positively selecting the test substance that provides a decrease in the replication capacity of the virus. Non-limiting examples of cell lines that can be suitable for the invention include but are not limited to BS-C-1, CV-1, Vero, Vero 76, Vero C1008, Vero 76, Cos-1, Cos-7, Huh7, FR11K-4, LLC-MK2 original, LLC-MK2 derivative, MDCK, RD, A549, MRC-5, KB, PER.C6, HEK-293 and CaCo-2 cells.

In some embodiments, the substances selected by the above mentioned screening method may be used in the treatment of SARS-Cov-2 infection (i.e. COVID-19). For example, the selected substances includes the reduction or amelioration of the progression, severity and/or duration of SARS-Cov-2 infections, or the amelioration of one or more symptoms (specifically, one or more discernible symptoms) of SARS-Cov-2 infections, resulting from the administration of at least one substance selected by the above mentioned screening method.

The invention will be further illustrated by the following figures and examples. However, these examples and figures should not be interpreted in any way as limiting the scope of the invention.

### FIGURES:

**Figure 1****:** sequence of SARS-CoV-2 nsp5-ppla/pplab 3CL (chymotrypsin-like) protease (3CLpro) catalytic domain (1-196) obtained from NCBI database (NC_045512.2)
**Figure 2****:** nucleotide sequence of SARS-CoV-2 3CLpro⁽¹⁻¹⁹⁶⁾ bacterial expression construct. The start codon is followed in frame by a 6-HIS tag, a GGGSGGG linker, FLAG- and GST-tags, a second GGGSGGG linker, a HRV 3C cleavage site and the 3CLpro⁽¹⁻¹⁹⁶⁾ catalytic domain. The 3CLpro⁽¹⁻¹⁹⁶⁾ catalytic domain has been codon optimized for bacterial expression in *E. coli.*
**Figure 3**: schematic representation of the SARS-CoV-2 3CLpro⁽¹⁻¹⁹⁶⁾ bacterial expression construct. The start codon is followed in frame by a 6-HIS tag, a GGGSGGG linker, FLAG- and GST-tags, a second GGGSGGG linker, a HRV 3C cleavage site and the 3CLpro⁽¹⁻¹⁹⁶⁾ catalytic domain.

### EXAMPLE 1: RAPID PRODUCTION OF SOLUBLE RECOMBINANT SARS-CoV-2 3CLpro CATALYTIC DOMAIN

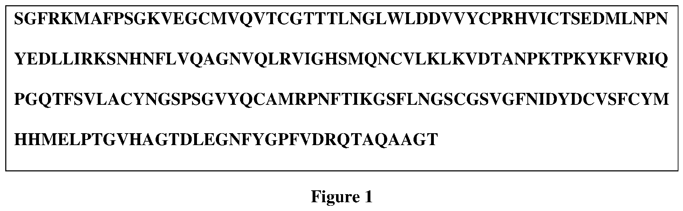

The coding sequence of SARS-CoV-2 nsp5-ppla/pplab 3CL (chymotrypsin-like) protease (3CLpro) was obtained from NCBI database (NC_045512.2) (Figure 1) and its catalytic domain (S1-T196) was *E.coli* codon-optimized for bacterial expression. The catalytic domain of 3CLpro (S1-T196) was chemically synthetized by Genecust (Boynes, France) and cloned into a bacterial expression vector, pET21. The catalytic domain was cloned in frame preceded by the start codon, a 6-Histidine (HIS) tag, a linker (GGGSGGG), a FLAG tag and a glutathione S-transferase (GST) tag. Following the GST tag is another linker (GGGSGGG) and a HRV 3C cleavage site which allows purification of untagged 3CLpro catalytic domain (Figures 2 and 3). GST folds rapidly into a stable and highly soluble protein upon translation, which allows inclusion of the GST tag to often promote greater expression and solubility of recombinant proteins than expression without the tag. The recombinant plasmid DNA was transformed into ClearColi BL21 (DE3) bacteria. Several bacterial clones were picked up, expanded and induced with ImM IPTG at 37°C during 4 hours. Bacterial culture were lyzed by sonication and centrifuged. Collected protein fractions (soluble, insoluble) were analyzed by SDS-PAGE, Coomassie blue staining showing repartition of proteins into the fractions. Soluble proteins were purified using the 6-HIS tag on a TALON Nickel affinity column and eluted with Imidazole gradient (0 to 500 mM). A buffer exchange against PBS was realized using PD-10 desalting column (Amersham).

Advantages of the construct:
- Production of high yields of highly soluble SARS-CoV-2 3CLpro catalytic domains;
- 3CLpro catalytic domain can either be used coupled to its tags or alone after cleavage by the HRV 3C protease;
- 3CLpro catalytic domain attached to its tags can be efficiently used for *in vitro* protease activity assay, allowing high-throughput screening of inhibitory compounds ;
- GST tag can be used for covalent functionalization of inert structures as nanoparticles, ELISA plaques or chromatography columns.

### REFERENCES:

Throughout this application, various references describe the state of the art to which this invention pertains. The disclosures of these references are hereby incorporated by reference into the present disclosure.
1. Chan, J.F.; Yuan, S.; Kok, K.H.; To, K.K.; Chu, H.; Yang, J.; Xing, F.; Liu, J.; Yip, C.C.; Poon, R.W., et al. A familial cluster of pneumonia associated with the 2019 novel coronavirus indicating person-to-person transmission: a study of a family cluster. Lancet 2020, 395, 514-523
2. Huang, C.; Wang, Y.; Li, X.; Ren, L.; Zhao, J.; Hu, Y.; Zhang, L.; Fan, G.; Xu, J.; Gu, X., et al. Clinical features of patients infected with 2019 novel coronavirus in Wuhan, China. Lancet 2020, 395, 497-506
3. Lu, R.; Zhao, X.; Li, J.; Niu, P.; Yang, B.; Wu, H.; Wang, W.; Song, H.; Huang, B.; Zhu, N., et al. Genomic characterisation and epidemiology of 2019 novel coronavirus: implications for virus origins and receptor binding. Lancet 2020, 395, 565-574,

## Claims

1. A recombinant fusion protein wherein the catalytic domain of the SAR-Cov-2 3CLpro that consists of the amino acid sequence that ranges from the amino acid residue (S) at position 1 to the amino acid residue (T) at position 196 in SEQ ID NO: 1 is fused to one or more expression tag(s).

2. The recombinant fusion protein of claim 1 wherein the expression tag is a GST tag.

3. The recombinant fusion protein of claim 1 wherein the catalytic domain of the SARS-Cov-2 3CLpro is fused either directly or via a linker at its C-terminal end to the N-terminal end of the expression tag, or at its N-terminal end to the C-terminal end of the expression tag.

4. The method of claim 1 wherein the linker consists of the amino acid sequence as set forth in SEQ ID NO:7.

5. The recombinant fusion protein of claim 1 that further comprises one or more purification tag.

6. The recombinant fusion protein of claim 5 that comprises a 6-Histidine (HIS) tag and a Flag tag.

7. The recombinant fusion protein of claim 1 that further comprises a cleavable sequence.

8. The recombinant fusion protein of claim 7 wherein the cleavable sequence is inserted between the catalytic domain of the 3CLpro and the expression tag.

9. The recombinant fusion protein of claim 7 wherein the cleavable sequence comprises the HRV 3C recognition site as set forth in SEQ ID NO:8.

10. The recombinant fusion protein of claim 1 wherein the catalytic domain of the 3CLpro is preceded by a 6-Histidine (HIS) tag, a linker (GGGSGGG), a FLAG tag and a glutathione S-transferase (GST) tag, and wherein the catalytic domain of the 3CLpro is fused to the GST tag by the linker (GGGSGGG) and a HRV 3C cleavage sequence.

11. The recombinant fusion protein of claim 1 that consists of an amino acid sequence having at least 90% of identity with the amino acid sequence as set forth in SEQ ID NO :9.

12. A polynucleotide that encodes for the fusion protein of claim 1.

13. The polynucleotide of claim 12 that comprises a nucleic acid sequence having at least 90% of identify with the nucleic acid sequence as set forth in SEQ ID NO: 10.

14. A vector that comprises the polynucleotide of claim 12.

15. A host cell comprising the polynucleotide of claim 12.

16. Use of the fusion protein of claim 1 for screening a plurality of test substances for their ability to inhibit the protease activity of the catalytic domain of the 3CLpro.
